# EUROPÄISCHE PATENTANMELDUNG

(11) **EP 1 269 938 A1**
(43) Veröffentlichungstag der Anmeldung: **02.01.2003**
(21) Anmeldenummer: 01115511.6
(22) Anmeldetag: 27.06.2001
(51) Int. Cl.: A61F 2/38

(54) **Gekoppelte Knieprothese mit Rotationslager**

(71) Anmelder: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(57) **Zusammenfassung**

Knieprothese bestehend aus einer Femurkomponente (1) mit starr miteinander verbundenen Kondylengleitflächen (20), einer Tibiakomponente (2) mit einem undrehbar mit ihr verbundenen Tibiaplateau (7), das mit den kondylären Gleitflächen (20) zusammenwirkende tibiale Gleitflächen (21) aufweist, und einer Koppelungseinrichtung (10), die mit der Femurkomponente (1) ein Beugelager (11) und mit der Tibiakomponente (2) ein Rotationslager (12) bildet, dessen Achse (12) gegenüber der Schienbeinrichtung (5) geneigt ist. Um eine günstigere Kraftverteilung in der Prothese und eine Rückstellkraft bei Rotation der Prothesenteile zu erreichen, ist die Anordnung so getroffen, daß die Normale (25) auf den in der Streckstellung hauptsächlich lastübertragenden mit den kondylären Gleitflächen (20) zusammenwirkenden Bereich (24) der tibialen Gleitflächen (21) in der Seitenansicht bzw. im Sagittalschnitt weniger gegenüber der Schienbeinrichtung (5) geneigt ist als die Achse (12) des Rotationslagers.

## Beschreibung

Es sind Knieprothesen bekannt, deren femorale und tibiale Komponenten unterschiedliche Bewegungsfreiheitsgrade zueinander aufweisen. Je geringer die verbliebene Stabilität des mit der Prothese auszurüstenden Knies ist, um so stärker muß die Prothese stabilisieren und um so geringer ist die Zahl der Freiheitsgrade, die der Relativbewegung zwischen den beiden Komponenten zugestanden werden kann, und umgekehrt. Die Beschränkung der Freiheitsgrade erreicht man durch eine Koppelungseinrichtung, die zwischen der femoralen und der tibialen Komponente wirkt. Die Erfindung bezieht sich gemäß dem Oberbegriff des Anspruchs 1 auf denjenigen Typ von Knieprothesen, der mit einer Koppelungseinrichtung ausgerüstet ist, die mit der Femurkomponente ein Beugelager und mit der Tibiakomponente ein Rotationslager bildet. Das Beugelager bestimmt die Bewegung der Komponenten um die Querachse bei der Beugung. Das Rotationslager, dessen Achse etwa parallel zur Schienbeinrichtung verläuft, ermöglicht eine gewisse Rotation um die Hochachse.

Die axialen Kräfte werden von den Kondylengleitflächen der femoralen Komponente auf damit zusammenwirkende tibiale Gleitflächen auf der Oberseite des Tibiaplateaus übertragen. Unter diesen ist zwischen zwei Gruppen zu unterscheiden. Bei den Prothesen der ersten Gruppe umfaßt das Rotationslager ein gegenüber der Tibiakomponente drehbares Plateau, dessen oberseitige Gleitfläche nur für die Beugebewegung mit den kondylären Gleitflächen der Femurkomponente zusammenwirkt (DE-B-2334265, DE-A-2636816, EP-A-716839, US-A-4888021, US-A-5370701). Bei den Prothesen der zweiten Gruppe ist das Tibiaplateau undrehbar mit der Tibiakomponente verbunden (US-A-5139521, EP-B-410237, EP-B-539654, EP-A-791343). Die Erfindung bezieht sich auf die zweite Gruppe. In diesem Fall vollzieht sich eine Relativbewegung zwischen den femoralen Kondylengleitflächen und den tibialen Gleitflächen nicht nur bei der Beugebewegung, sondern auch bei der Rotationsbewegung. Damit bei der Rotationsbewegung beide femoralen Kondylengleitflächen den kraftübertragenden Kontakt mit den zugehörigen Bereichen der tibialen Gleitfläche behalten, hält man es im Stand der Technik für erforderlich, daß diese Bereiche der tibialen Gleitfläche im wesentlichen lotrecht zu der Rotationsachse orientiert sind. Das gilt zumindest für denjenigen Beugebereich, in welchem die Lastübertragung hauptsächlich stattfindet. Das ist im allgemeinen die Streckstellung oder eine der Streckstellung nahe Beugestellung. Die Bedingung der im wesentlichen lotrechten Orientierung der tibialen Gleitflächen in diesem lastübertragenden Bereich gegenüber der Rotationsachse wurde bislang auch dann beachtet, wenn die Richtung der Rotationsachse gegenüber der Schienbeinachse geneigt ist, so daß sie nicht auf das Sprunggelenk, sondern auf die Fußfläche gerichtet ist (EP-B-410237). Die tibiale Gleitfläche ist dabei ebenso geneigt wie die Rotationsachse. Sie fällt nach hinten ab. Dies hat erhebliche Nachteile für die Beanspruchung des Rotationslagers. Die Neigung der tibialen Gleitfläche nach hinten führt nämlich zu einer Horizontalkraftkomponente, die das Rotationslager mit einem Moment um die Horizontalachse belastet und damit erhöhtem Verschleiß aussetzt.

Die Erfindung will diesen Nachteil vermeiden und erreicht dies durch die Merkmale des Anspruchs 1.

Obwohl die Rotationssache geneigt verläuft, wird die tibiale Gleitfläche erfindungsgemäß nicht mit einer entsprechenden Neigung versehen, was kinematisch widersprüchlich erscheint, weil es im Falle der Rotation die symmetrische Kraftübertragung über beide Kondylenflächen ausschließt. Erreicht wird dadurch zum einen, daß die erwähnte Horizontalkraftkomponente und die von dieser verursachte Mehrbeanspruchung des Rotationslagers vermieden wird. Zum anderen hat die Erfindung den Vorteil, daß eine Rotationsbewegung der Prothesenkomponenten stets verbunden ist mit der Erzeugung einer rückstellenden Kraft. Während der Rotation wandert einer der beiden Kontaktpunkte der femoralen Kondylen auf der tibialen Gleitfläche nach vorne und der andere nach hinten. Da die tibiale Gleitfläche nicht lotrecht zur Rotationsachse steht, gewinnt einer der beiden Kondylenkontakte während dieser Verschiebung an Höhe gegenüber dem Rotationslager im Vergleich zu dem vorherigen Zustand. Sein Bestreben, unter der Last zurückzukehren in den tiefer gelegenen, vorherigen Zustand, erzeugt die Rückstellkraft.

Bevorzugt wird eine Ausführung, bei welcher die tibiale Gleitfläche etwa lotrecht zur Schienbeinrichtung verläuft.

Genauer gesagt, ist die Richtung der Normalen auf die tibiale Gleitfläche parallel zur Schienbeinrichtung. Dabei kommt es auf diejenige Stelle der tibialen Gleitfläche an, an welcher die Lastübertragung von der kondylären Gleitfläche auf die Tibiagleitfläche im gestreckten Zustand der Prothese hauptsächlich stattfindet. Der Erfindungsgedanke wird aber auch dann verwirklicht, wenn die tibiale Gleitfläche schwach geneigt verläuft, insbesondere wenn der Winkel zwischen der genannten Normalen und der Schienbeinrichtung nicht mehr als halb so groß ist wie der Winkel zwischen der Achse des Rotationslagers und der Schienbeinrichtung.

Die Tatsache, daß durch die Winkeldifferenz zwischen der Richtung des Rotationslagers und der genannten Normalen eine Rückstellkraft auf die rotierten Prothesenkomponenten ausgeübt wird, besagt nicht, daß auf weitere Mittel zur Erzeugung einer solchen Rückstellkraft verzichtet werden muß. Insbesondere kann zwischen den beiden Bereichen der tibialen Gleitfläche, die mit den beiden kondylären Gleitflächenteilen zusammenwirken, eine erhöhte Mittelrippe vorgesehen sein, wie es bekannt ist (DE 2744710).

Die Erfindung wird im folgenden näher unter Bezugnahme auf die Zeichnung erläutert, die ein vorteilhaftes Ausführungsbeispiel veranschaulicht. Es zeigen:
- Fig. 1: eine Dorsalansicht,
- Fig. 2: eine Schnittansicht und
- Fig. 3: eine Seitenansicht der Prothese.

Die Prothese umfaßt einen femoralen Teil 1 und einen tibialen Teil 2, die durch Dorne 3 bzw. 4 im Oberschenkelknochen bzw. im Schienbein zu verankern sind. Die Richtung 5 des Dorns 4 gibt die Schienbeinrichtung an. Die Last wird vom femoralen Teil 1 auf den tibialen Teil 2 in jeder Beugestellung durch femorale Kufen 6 und ein Tibiaplateau 7 übertragen. Zwecks Stabilisierung sind der femorale und der tibiale Teil durch einen Zwischenteil 10 miteinander verbunden, der als Koppelungseinrichtung mit dem femoralen Teil 1 ein Scharnier bildet, dessen Achse 11 mit der Beugeachse übereinstimmt und mit dem tibialen Teil 2 ein Rotationslager mit Rotationsachse 12 bildet. Das Rotationslager besteht aus einem Zapfen 15 des Zwischenteils 10 und einer Bohrung 13 im tibialen Teil, die eine Gleitbuchse 14, beispielsweise aus Polyethylen enthält, die den Zapfen 15 gleitpassend aufnimmt. Die Rotationsachse 12 schließt mit der Schienbeinrichtung 5 der Prothese in der Sagittalebene einen Winkel α ein, der im dargestellten Beispiel bei 9° (im allgemeinen zwischen 4° und 15°) liegt.

Die Kufen 6 des femoralen Teils treten an die Stelle der natürlichen Kondylen. Die von ihnen gebildeten Gleitflächen 20 werden deshalb als kondyläre Gleitflächen bezeichnet. Sie können in der Seitenansicht nach einem Kreisbogen geformt sein. In diesem Fall fällt ihre Krümmungsachse mit der Beugeachse 11 zusammen. Sie können auch zur besseren Annäherung an die natürlichen Verhältnisse polyzentrisch ausgebildet sein.

Das Tibiaplateau 7 ist starr auf einer Platte 17 des Tibiateils gehalten. Sie ist darauf auch vorzugsweise gegen Abheben (beispielsweise durch Schrauben) gesichert. Sie enthält einen Hinterschnitt 18, der im Zusammenwirken mit einem Kragen 19 des Zwischenteils 10 das Verbleiben des Zapfens 12 im Rotationslager sichert.

Das Tibiaplateau 7 bildet oberseitig eine tibiale Gleitfläche 21. Für jede Kondylengleitfläche 20 bildet sie einen Gleitflächenbereich. Dazwischen bildet das Tibiaplateau einen firstartig erhöhten Bereich 22, der in die interkondyläre Nut 23 des femoralen Teils hineinragt.

Die tibiale Gleitfläche 21 des Tibiaplateaus 7 ist in der Seitenansicht oder im Sagittalschnitt zweckmäßigerweise konkav gemuldet, um sich mehr oder weniger der Form der kondylären Gleitflächen 20 anzunähern. Dadurch wird die Flächenpressung vermindert. Eine vollständige Kongruenz ist zwar möglich, aber in den meisten Fällen weder erforderlich noch erwünscht. Hingegen ist im Frontalschnitt weitgehende Formübereinstimmung der kondylären und tibialen Gleitflächen 20, 21 erwünscht, wobei vorausgesetzt sind, daß sie ihre neutrale Stellung in bezug auf die Rotationsachse 12 einnehmen, die dem Streckzustand des Beins entspricht.

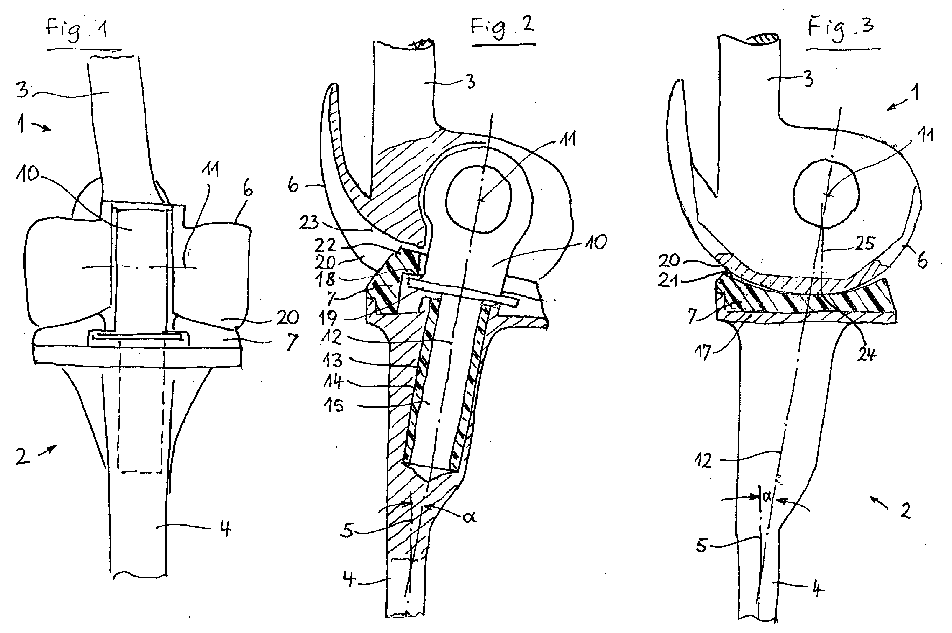

Wenn das Tibiaplateau 7 fest am Tibiateil 2 angeordnet ist, wird die Relativstellung der kondylären und tibialen Gleitflächen 20, 21 durch die Beugeachse 11 bestimmt. Wenn der Krümmungsradius der tibialen Gleitfläche 21 größer ist als derjenige der kondylären Gleitfläche 20, so sind die geometrischen Verhältnisse so gewählt, daß der theoretischgeometrische Berührungspunkt im nicht rotierten Zustand der Prothesenteile an einer Stelle der tibialen Gleitfläche liegt, deren Normale (d.h. eine lotrecht auf der Fläche der betreffenden Stelle stehende Linie) etwa parallel zur Schienbeinrichtung 5 verläuft. Diese Stelle und die zugehörige Normale sind in Fig. 3 mit den Ziffern 24 und 25 bezeichnet.

Wenn (abweichend von der dargestellten Ausführungsform) die Krümmungsradien der kondylären und tibialen Gleitflächen 20, 21 im Sagittalschnitt gleich sind, verteilt sich die Lastübertragung nicht über die gesamte theoretische Berührungsfläche. Vielmehr bildet sich ebenfalls eine Stelle der hauptsächlichen Kraftübertragung heraus. Diese hat im allgemeinen in der Streck- und Standstellung etwa horizontale Lage. Auch dafür gilt also, daß die Normale etwa parallel zur Schienbeinrichtung verläuft.

Wenn (abweichend von der dargestellten Ausführungsform) das Tibiaplateau 7 gegenüber dem Tibiateil 1 auf einer Führungsebene während der Beugebewegung vor- und zurückbewegbar ist, stellt sich das Plateau jeweils so ein, daß die tibiale Gleitfläche im Punkt der stärksten Kraftübertragung etwa parallel zu der Führungsebene des Tibiaplateaus verläuft. Die Normale auf den hauptsächlich lastübertragenden Bereich steht somit lotrecht zu der Führungsfläche.

In allen diesen Fällen verlangt die Erfindung, daß die Konstruktion so gestaltet ist, daß die Normale auf die hauptsächlich lastübertragende Stelle in der Seitenansicht weniger gegenüber der Schienbeinrichtung geneigt ist als die Achse des Rotationslagers. Wenn man sich die Schienbeinrichtung vertikal vorstellt, soll also die tibiale Gleitfläche an dieser Stelle etwa horizontal sein.

Wenn die Prothesenkomponenten 1 und 2 in bezug auf die Achse 12 unverdreht sind (neutrale Stellung), wie es in der Streckstellung in der Regel der Fall ist, liegen beide kondylären Gleitflächen kraftübertragend auf den zugehörigen Bereichen der tibialen Gleitfläche auf. Falls Rotation um die Achse 12 zwischen den Prothesenteilen 1 und 2 und den Gleitflächen 20 und 21 stattfindet, ergibt sich eine relative Verschiebung der bei Punkt 24 aufeinanderliegenden Gleitflächenbereiche in Richtung vor bzw. zurück. Wenn die Normale auf diesen Bereich parallel zur Rotationsachse 12 verlaufen würde, wie es bekannt ist, ergäbe sich dabei keine wesentliche Höhenänderung des betreffenden Punkts der kondylären Gleitfläche 20 in bezug auf das Rotationslager. Da aber gemäß der Erfindung die Normale 25 auf diesen Bereich eine andere Richtung hat als die Rotationsachse 12, sind die Gleitflächen 20, 21 an der betreffenden Stelle gegenüber der Umfangsrichtung geneigt. Daraus folgt, daß auf einer Kondylenseite ein Anheben der kondylären Gleitfläche 20 im Verhältnis zur Tibiakomponente der Prothese erzwungen wird. Unter der Last strebt die Anordnung daher zurück in die neutrale Rotationsstellung.

Die Erfindung hat ferner den Vorteil, daß die Richtung der Normalen 25 in den meisten Belastungsfällen etwa mit der Lastrichtung übereinstimmt. Die Entstehung quer dazu verlaufender Kräfte und daraus resultierender Biegemomente auf das Rotationslager 13, 14, 15 bleiben daher geringer als es der Fall wäre, wenn der betreffende Bereich der Gleitflächen ebenso geneigt wäre wie die Rotationsachse.

## Patentansprüche

1. Knieprothese bestehend aus einer Femurkomponente (1) mit starr miteinander verbundenen kondylären Gleitflächen (20), einer Tibiakomponente (2) mit einem undrehbar mit ihr verbundenen Tibiaplateau (7), das mit den kondylären Gleitflächen (20) zusammenwirkende, tibiale Gleitflächen (21) aufweist, und einer Koppelungseinrichtung (10), die mit der Femurkomponente (1) ein Beugelager (11) und mit der Tibiakomponente (2) ein Rotationslager (12 bis 14) bildet, dessen Achse (12) gegenüber der Schienbeinrichtung (5) geneigt ist, **dadurch gekennzeichnet, daß** die Normale (25) auf den in der Streckstellung hauptsächlich lastübertragend mit der kondylären Gleitfläche (20) zusammenwirkenden Bereich (24) der tibialen Gleitflächen (21) in der Seitenansicht bzw. im Sagittalschnitt weniger gegenüber der Schienbeinrichtung (5) geneigt ist, als die Achse (12) des Rotationslagers.

2. Knieprothese nach Anspruch 1, **dadurch gekennzeichnet, daß** der Winkel zwischen der genannten Normalen (25) und der Schienbeinrichtung (5) nicht mehr als halb so groß ist wie der Winkel (α) zwischen der Achse (12) des Rotationslagers und der Schienbeinrichtung (5).

3. Knieprothese nach Anspruch 2, **dadurch gekennzeichnet, daß** die genannte Normale (25) etwa parallel zur Schienbeinrichtung verläuft.

4. Knieprothese nach einem der Ansprüche 1 bis 3, **dadurch gekennzeichnet, daß** die tibiale Gleitfläche (21) im interkondylären Bereich (22) erhöht ist.
